# EUROPEAN PATENT APPLICATION

(11) **EP 2 366 681 A1**
(43) Date of publication of application: **21.09.2011**
(21) Application number: 09826058.1
(22) Date of filing: 06.11.2009
(51) Int. Cl.: C07C 1/04, B01J 23/889, B01J 35/10, C07C 9/02, C07C 9/04, C07C 9/14, C07C 9/22, C07C 11/02, C07C 29/156, C07C 31/04, C07C 31/08, C07C 31/10, C07C 31/12, C07B 61/00

(54) **METHOD FOR MANUFACTURING UNSATURATED HYDROCARBON AND OXYGENATED COMPOUND, CATALYST, AND MANUFACTURING METHOD THEREFOR**

(30) Priority: 11.11.2008 JP 2008288797
(71) Applicant: JX Nippon Oil & Energy Corporation, Chiyoda-ku Tokyo 100-8162 (JP); National University Corporation University Of Toyama, Toyama-shi, Toyama 930-8555 (JP)
(72) Inventor: TSUBAKI, Noritatsu, Toyama-shi Toyama 930-8555 (JP); AIDA, Fuyuki, Yokohama-shi Kanagawa 231-0815 (JP)
(74) Representative: Grünecker, Kinkeldey, Stockmair & Schwanhäusser Anwaltssozietät
(86) International application number: PCT/JP2009/068967
(87) International publication number: WO 2010/055808

(57) **Abstract**

The method for manufacturing an unsaturated hydrocarbon and an oxygen-containing compound according to the present invention comprises: a first step of dispersing a catalyst in poly-α-olefin and reducing the catalyst with carbon monoxide or synthesis gas, wherein the catalyst is prepared by supporting iron on a support containing manganese and having an average pore size of 2 to 100 nm; and a second step of bringing the catalyst after reduction in the first step into contact with synthesis gas under the conditions of a reaction temperature of 100 to 600°C and a reaction pressure of 0.1 to 10 MPa to obtain a reaction product containing an unsaturated hydrocarbon and an oxygen-containing compound.

## Description

### Technical Field

The present invention relates to a method for manufacturing an unsaturated hydrocarbon and an oxygen-containing compound, and also relates to a catalyst and a method for manufacturing thereof.

### Background Art

The Fischer-Tropsch synthesis (FT synthesis) is known as a method for synthesizing hydrocarbons from synthesis gas (a mixture of carbon monoxide and hydrogen).

Hydrocarbon synthesis from synthesis gas has been aiming mostly at saturated hydrocarbons, as typified by gas-to-liquids (GTL). Such saturated hydrocarbons are used as fuel or lubricating oil through various steps, such as hydrocracking and isomerization. Note that, in this case, unsaturated hydrocarbons and oxygen-containing compounds can also be produced simultaneously with the production of saturated hydrocarbons, but the selectivity of unsaturated hydrocarbons and oxygen-containing compounds is very low. Therefore, these unsaturated hydrocarbons and oxygen-containing compounds are commonly hydrogenated for use as saturated hydrocarbons.

On the other hand, a method for manufacturing unsaturated hydrocarbons and oxygen-containing compounds as the target substances from synthesis gas has been studied because unsaturated hydrocarbons, that is, olefins, and oxygen-containing compounds typified by alcohols are useful as raw materials for chemicals.

For example, Patent Literatures 1 and 2 disclose the FT reaction aiming at producing olefins in high yield using an iron-based catalyst in which a manganese-based compound is used as a support.

Further, Patent Literature 3 discloses the FT reaction using a catalyst in which iron, copper, and potassium are supported on a silica porous support.

Furthermore, Patent Literatures 4 and 5 disclose a method for manufacturing olefins from synthesis gas using a catalyst in which ruthenium is supported on a manganese-based compound as a support.

### Citation List

### Patent Literature

Patent Literature 1: Japanese Examined Patent Application Publication No. 56-48491
Patent Literature 2: US 4,177,203 A
Patent Literature 3: Japanese Patent Application Laid-Open No. 2006-297286
Patent Literature 4: US 4,206,134 A
Patent Literature 5: Japanese Examined Patent Application Publication No. 3-70691

### Summary of Invention

### Technical Problem

However, the catalysts or methods disclosed in the above Patent Literatures 1 to 5 are not necessarily satisfactory in terms of the conversion (CO conversion) of carbon monoxide in synthesis gas, the selectivity of unsaturated hydrocarbons and oxygen-containing compounds, and the like, but there is room for improvement so that these catalysts or methods may be accepted for practical utilization.

The present invention has been made in the light of the above-described circumstances, and it is an object of the present invention to provide a method capable of achieving high CO conversion and high selectivity of unsaturated hydrocarbons and oxygen-containing compounds in the FT reaction, and a catalyst used in this method and a method for manufacturing thereof.

### Solution to Problem

As a result of extensive studies to achieve the above object, the present inventors have found that, when the FT reaction is performed under a specific condition using a catalyst in which iron is supported on a support containing manganese and having a specific average pore size, significantly high CO conversion is given, the reaction is very highly selective to olefin and alcohol, and as a result, the sum of the selectivity of olefin and the selectivity of alcohol is significantly high. The present invention has been completed on the basis of these findings.

Specifically, the present invention provides a method for manufacturing an unsaturated hydrocarbon and an oxygen-containing compound according to the following (1) to (4), a catalyst according to the following (5) to (7), and a method for manufacturing the catalyst according to the following (8). Note that the term "synthesis gas" as used in the present invention means a mixed gas of carbon monoxide and hydrogen.
(1) A method for manufacturing an unsaturated hydrocarbon and an oxygen-containing compound, characterized in that the method comprises: a first step of dispersing a catalyst in poly-α-olefin and reducing the catalyst with carbon monoxide or synthesis gas containing carbon monoxide and hydrogen, wherein the catalyst is prepared by supporting iron on a support containing manganese and having an average pore size of 2 to 100 nm; and a second step of bringing the catalyst after reduction in the first step into contact with synthesis gas under the conditions of a reaction temperature of 100 to 600°C and a reaction pressure of 0.1 to 10 MPa to obtain a reaction product containing an unsaturated hydrocarbon and an oxygen-containing compound.
(2) The method for manufacturing an unsaturated hydrocarbon and an oxygen-containing compound according to (1), characterized in that the reaction temperature in the second step is kept within the range of 280°C plus or minus 20°C.
(3) The method for manufacturing an unsaturated hydrocarbon and an oxygen-containing compound according to (1) or (2), characterized in that the catalyst is a catalyst prepared by further supporting copper and/or potassium on the support.
(4) The method for manufacturing an unsaturated hydrocarbon and an oxygen-containing compound according to any of (1) to (3), characterized in that the support has an average pore size of 2 to 50 nm.
(5) A catalyst characterized in that the catalyst is prepared by supporting iron on a support containing manganese and having an average pore size of 2 to 100 nm.
(6) The catalyst according to (5), characterized in that the catalyst is prepared by further supporting copper and potassium on the support.
(7) The catalyst according to (5) or (6), characterized in that the support has an average pore size of 2 to 50 nm.
(8) A method for manufacturing a catalyst, characterized in that the method comprises: a third step of mixing a support containing manganese and having an average pore size of 2 to 100 nm with a solution containing iron; a fourth step of decompressing and drying the mixture obtained in the third step to allow the iron to adhere to the pores of the support to obtain a catalyst precursor; and a fifth step of calcining the catalyst precursor obtained in the fourth step.

### Advantageous Effects of Invention

The method for manufacturing an unsaturated hydrocarbon and an oxygen-containing compound according to the present invention and the catalyst of the present invention can achieve high CO conversion and high selectivity of unsaturated hydrocarbons and oxygen-containing compounds in the FT reaction. Further, the method for manufacturing a catalyst according to the present invention can effectively provide the catalyst of the present invention having excellent properties as described above.

### Description of Embodiments

Hereinafter, preferred embodiments of the present invention will be described in detail.

The catalyst of the present invention is a catalyst prepared by supporting iron on a support containing manganese and having an average pore size of 2 to 100 nm.

The support which constitutes the catalyst of the present invention contains manganese (Mn) as an essential element, but in addition to Mn, it may further contain an element selected from the elements of Groups IA, IIA, IIIB, IVB, IIIA, and IVA of the Periodic Table.

Further, the support has an average pore size of 2 to 100 nm, preferably 2 to 50 nm, as described above. When the average pore size is less than 2 nm, the pores will be liable to get blocked during the FT synthesis, and it will become difficult to maintain a suitable catalytic reaction. When the average pore size exceeds 100 nm, the surface area per unit weight will be significantly small, and it will become difficult to sufficiently ensure the amount of supported metal such as iron.

Note that the term "average pore size" as used in the present invention means the value determined by a nitrogen adsorption method using Quanta Chrome Autosorb-1 manufactured by Yuasa Ionics Co., Ltd. which is an adsorption measuring apparatus.

The specific surface area of the support used in the present invention is not particularly limited, but it is preferred that the specific surface area by the BET adsorption method be in the range of 100 to 1000 m²/g. Further, the pore volume of the support is not particularly limited, but is preferably in the range of 0.2 to 2.0 ml/g.

The shape of the support is not particularly limited, but a shape suited to the process to be used may be appropriately selected from a shape such as a spherical shape, a crushed shape, and a cylindrical shape.

In the catalyst of the present invention, it is possible to combine a support which does not contain Mn, such as silica, silica-alumina, alumina, and titania, with the support as described above.

Any Fe-compound such as inorganic salts and organic complexes of Fe can be used as a Fe-compound used for supporting iron (Fe) on the above support. Among them, sulfates, nitrates, organic acid salts, and chlorides are suitably used. Specific examples thereof include ferrous sulfate, ferric sulfate, ferrous nitrate, ferric nitrate, ferrous chloride, ferric chloride, iron carbonyl, potassium ferrocyanide, potassium ferricyanide, and iron acetylacetonate (Fe(acac)₂, Fe(acac)₃).

The catalyst of the present invention may further contain metal other than Fe as a supported metal. In particular, when copper (Cu) and/or potassium (K) are supported on the above support in addition to Fe, the resulting catalyst is preferred in terms of catalytic activity. A compound used for supporting Cu and K is not particularly limited. For example, any compound such as inorganic salts and organic complexes of Cu can be used as a Cu compound. Among them, sulfates, nitrates, organic acid salts, and chlorides are suitably used. Specific examples include copper sulfate, copper nitrate, copper chloride, and copper acetate.

The amount of the metal supported on the support in the present invention is not particularly limited, but the amount of Fe supported is, to the support, preferably 3 to 50% by weight, more preferably 5 to 40% by weight, further preferably 10 to 30% by weight, most preferably 15 to 25% by weight. When Cu is supported, the amount of Cu supported is preferably 0.5 to 6% by weight, more preferably 1 to 4% by weight, to the support.

The method for supporting Fe or the like on the support can be appropriately selected from conventional methods such as an impregnation method and an ion exchange method. As a particularly preferred method, an impregnation method can be mentioned. As a particularly preferred method in the impregnation method, the Incipient Wetness method can be mentioned. When a plurality of metals is impregnated, both simultaneous impregnation and sequential impregnation can be selected, but simultaneous impregnation is preferred.

The catalyst of the present invention can be suitably obtained by the method for manufacturing the catalyst of the present invention comprising the following 3 steps:
(A-1) a step of mixing a support containing Mn and having an average pore size of 2 to 100 nm with a solution containing Fe;
(A-2) a step of decompressing and drying the mixture obtained in the above step (A-1) to allow the Fe to adhere to the pores of the support to obtain a catalyst precursor; and
(A-3) a step of calcining the catalyst precursor obtained in the above step (A-2).

A solvent can be used in the step (A-1). Any solvent can be used without limitation as long as it can disperse a support containing Mn and can dissolve at least a Fe compound. Specific examples thereof include water, ketone compounds such as acetone, and alcoholic solvents such as methanol, ethanol, and isopropyl alcohol. Generally, the processing in the step (A-1) is satisfactorily performed at ordinary temperature, but it is preferably performed at about 60°C using ultrasonic vibration. Note that, for supporting Cu, K, and the like in addition to Fe, these metals may be added to the solution containing Fe.

The decompression and drying in the step (A-2) is preferably performed at a pressure of 100 kPa or less and a temperature of 40°C or higher. Agitation is preferably employed in order to uniformly adhere metal components such as Fe to pores.

The calcining in the step (A-3) is preferably performed at a temperature of 100°C or higher in an air atmosphere. More preferably, the calcining is performed at 120°C for 12 hours or more in an air atmosphere.

The method for manufacturing an unsaturated hydrocarbon and an oxygen-containing compound according to the present invention is a method in which the above catalyst of the present invention is used, and the method comprises the following two steps:
(B-1) a step of dispersing the catalyst of the present invention in poly-α-olefin (PAO) and reducing the catalyst with carbon monoxide or synthesis gas containing carbon monoxide and hydrogen (hereinafter referred simply to as "synthesis gas"); and
(B-2) a step of bringing the catalyst after reduction in the above step (B-1) into contact with synthesis gas under the conditions of a reaction temperature of 100 to 600°C and a reaction pressure of 0.1 to 10 MPa to obtain a reaction product containing an unsaturated hydrocarbon and an oxygen-containing compound.

In the (B-1) step, it is preferable to introduce the catalyst of the present invention into a reactor and disperse it in the PAO to form a slurry. The activity of the catalyst of the present invention can be further increased by employing such a slurry form and reducing the catalyst with synthesis gas (a mixture of carbon monoxide and hydrogen, in which the ratio may be arbitrary) or carbon monoxide in the reactor.

The ratio of the catalyst and the PAO is basically arbitrary, but the PAO is preferably used in the range of 1 ml to 10 L, to 1 g of the catalyst. Further, the reduction temperature is preferably in the range of 100 to 400°C.

The PAO to be used preferably has a boiling point of 300°C or higher. When the catalyst is reduced particularly with carbon monoxide using such PAO, the production ratio of an oxygen-containing compound to an unsaturated hydrocarbon in the step (B-2) tends to be significantly higher. In other words, the ratio of unsaturated hydrocarbon/oxygen-containing compound tends to be significantly lower.

The step (B-1) is the in-situ reduction method for activating a catalyst in the system, and the FT synthesis is performed in the step (B-2) following this step (B-1). The reaction temperature in the step (B-2) is selected from the range of from 100 to 600°C. When the reaction temperature is less than 100°C, the activity will be insufficient, which extremely reduces the conversion, and when it exceeds 600°C, decomposition of a reaction product and PAO will take place easily. Thus, the reaction temperature is preferably in the range of 220 to 340°C, more preferably in the range of 280°C plus or minus 20°C. By employing the preferred reaction temperature within the range as described above, the unsaturated hydrocarbon and the oxygen-containing compound can be produced such that the total of these compounds are 25% or more, and the ratio of unsaturated hydrocarbon/oxygen-containing compound can be selected in the range of 0.1 to 3.0. As a result, olefin or alcohol useful as a chemical can be selectively produced.

The reaction pressure in the step (B-2) is selected from the range of 0.1 to 10 MPa, and is preferably in the range of 0.5 to 5 MPa. When the reaction pressure is less than 0.1 MPa, the contact probability between the catalyst dispersed in PAO and synthesis gas will be reduced, thereby causing reduction in the reactivity. However, pressurization exceeding 10 MPa is not preferred because it may be an excessive pressurization, which requires excessive facilities.

In the above method for manufacturing an unsaturated hydrocarbon and an oxygen-containing compound according to the present invention, precipitation of wax on a catalyst surface does not take place easily and heat of reaction can be easily removed. Therefore, this process is most preferably applied to a slurry bed process which is advantageous to industrialization, but it can also be used in a fixed bed process or a fluidized bed process which are conventionally known.

Note that, in a slurry bed process, the diffusion of reaction raw materials to the catalyst surface is an important factor. On the other hand, since the catalyst particles will rub against each other or the catalyst will rub against a reactor wall in the reactor, mechanical strength for the catalyst is also required. The catalyst of the present invention can achieve these requirements at a high level.

### Examples

Hereinafter, the present invention will be more specifically described with reference to Examples and Comparative Examples, but the present invention is not at all limited to the following Examples. Note that the unit "%" for CO conversion, yield, and selectivity in the following Examples means % by mole.

### Example 1

A crushed manganese oxide support having a K-content of 8% by weight (trade name: N-190, manufactured by Sued-Chemie Catalysts Japan, Inc., having a BET specific surface area of 398 m²/g, pore volume of 0.70 ml/g, and an average pore size of 10.1 nm) was classified to a size range of 20 to 40 mesh. The manganese oxide support in an amount of 5 g was impregnated with an aqueous solution containing Fe(NO₃)₃·9H₂O in an amount corresponding to 20% by weight of the manganese oxide in terms of metallic iron by the Incipient Wetness method using ultrasonic vibration. The resulting mixture was subjected to vacuum drying at 65°C for 6 hours, dried at 120°C for 12 hours, heated from room temperature to 400°C at 2°C/min, and calcined for 2 hours at 400°C.
The catalyst in an amount of 1 g prepared in this way was introduced into a slurry type reactor, and thereto were added 20 ml of PAO (poly-α-olefin). The reactor was controlled to a temperature of 280°C and a pressure of 1.0 MPa, and thereto was passed through a synthesis gas of H₂/CO=1/1 at 10 gh/mol for 6 hours to reduce the catalyst. The FT reaction was performed under the same conditions as in the reduction, and a sample was collected after the lapse of 10 hours and determined for products by GC using trans-decalin and 1-octanol as standard substances.
The CO conversion was 80%, and the yield of each product was as follows: CO₂ (47%), methane (3%), an oxygen-containing compound (8%), olefin (24%), and paraffin (8%). The total yield of the unsaturated hydrocarbon and the oxygen-containing compound was 32%, and the ratio of unsaturated hydrocarbon/oxygen-containing compound was 2.9.
In the hydrocarbon excluding the oxygen-containing compound, the olefin/paraffin ratio (hereinafter referred to as O/P) for C₂ to C₄ was 5; the O/P for C₅ to C₁₁ was 3; and the O/P for C₁₂ or higher was 1.
In the hydrocarbon excluding the oxygen-containing compound, the selectivity of each component was as follows: methane (9%), C₂ to C₄ (38%), C₅ to C₁₁ (47%), and C₁₂ or higher (6%).
Further, the selectivity of the main compounds, when all the oxygen-containing compounds were defined as 100, was as follows: methanol (12%), ethanol (49%), 1-propanol (13%), and 1-butanol (7%).

### Example 2

A catalyst was prepared in the same manner as in Example 1 except that Cu(NO₃)₂·3H₂O in an amount corresponding to 3% by weight of manganese oxide in terms of metallic copper was used as a supported metal in addition to Fe(NO₃)₃·9H₂O. The resulting catalyst was used to perform the FT reaction.
The CO conversion was 89%, and the yield of each product was as follows: CO₂ (44%), methane (6%), an oxygen-containing compound (15%), olefin (25%), and paraffin (10%). The total of the unsaturated hydrocarbon and the oxygen-containing compound was 40%, and the ratio of unsaturated hydrocarbon/oxygen-containing compound was 1.7.
In the hydrocarbon excluding the oxygen-containing compound, the O/P for C₂ to C₄ was 5; the O/P for C₅ to C₁₁ was 4; and the O/P for C₁₂ or higher was 1.
In the hydrocarbon excluding the oxygen-containing compound, the selectivity of each component was as follows: methane (9%), C₂ to C₄ (38%), C₅ to C₁₁ (39%), and C₁₂ or higher (15%).
Further, the selectivity of the main compounds, when all the oxygen-containing compounds were defined as 100, was as follows: methanol (7%), ethanol (57%), 1-propanol (15%), and 1-butanol (7%).

### Example 3

The FT reaction was performed in the same manner as in Example 2 except that the reaction temperature was changed to 300°C.
The CO conversion was 93%, and the yield of each product was as follows: CO₂ (44%), methane (1%), an oxygen-containing compound (41%), olefin (6%), and paraffin (7%). The total yield of the unsaturated hydrocarbon and the oxygen-containing compound was 47%, and the ratio of unsaturated hydrocarbon/oxygen-containing compound was 0.1.
In the hydrocarbon excluding the oxygen-containing compound, the O/P for C₂ to C₄ was 5; the O/P for C₅ to C₁₁ was 4; and the O/P for C₁₂ or higher was 3.
In the hydrocarbon excluding the oxygen-containing compound, the selectivity of each component was as follows: methane (9%), C₂ to C₄ (37%), C₅ to C₁₁ (40%), and C₁₂ or higher (15%).

### Example 4

The FT reaction was performed in the same manner as in Example 2 except that the reaction temperature was changed to 260°C.
The CO conversion was 60%, and the yield of each product was as follows: CO₂ (45%), methane (2%), an oxygen-containing compound (7%), olefin (19%), and paraffin (6%). The total yield of the unsaturated hydrocarbon and the oxygen-containing compound was 25%, and the ratio of unsaturated hydrocarbon/oxygen-containing compound was 2.7.
In the hydrocarbon excluding the oxygen-containing compound, the O/P for C₂ to C₄ was 5; the O/P for C₅ to C₁₁ was 3; and the O/P for C₁₂ or higher was 1.
In the hydrocarbon excluding the oxygen-containing compound, the selectivity of each component was as follows: methane (8%), C₂ to C₄ (34%), C₅ to C₁₁ (43%), and C₁₂ or higher (16%).

### Example 5

A crushed manganese oxide support having a K-content of 3% by weight (trade name: MN-280, manufactured by Sued-Chemie Catalysts Japan, Inc., having a BET specific surface area of 381 m²/g, pore volume of 0.55 ml/g, and an average pore size of 4.7 nm) was classified to a size range of 20 to 40 mesh. The manganese oxide support in an amount of 5 g was impregnated simultaneously with an aqueous solution containing Fe(NO₃)₃·9H₂O in an amount corresponding to 20% by weight of the manganese oxide in terms of metallic iron and Cu(NO₃)₂·3H₂O in an amount corresponding to 3% by weight of manganese oxide in terms of metallic copper by the Incipient Wetness method using ultrasonic vibration. The resulting mixture was subjected to vacuum drying at 65°C for 6 hours, dried at 120°C for 12 hours, heated from room temperature to 400°C at 2°C/min, and calcined for 2 hours at 400°C.
The catalyst in an amount of 1 g prepared in this way was introduced into a slurry type reactor, and thereto were added 20 ml of PAO (poly-α-olefin). The reactor was controlled to a temperature of 280°C and a pressure of 1.0 MPa, and thereto was passed through a synthesis gas of H₂/CO=1/1 at 10 gh/mol for 6 hours to reduce the catalyst. The FT reaction was performed under the same conditions as in the reduction, and a sample was collected after the lapse of 10 hours and determined for products by GC using trans-decalin and 1-octanol as standard substances.
The CO conversion was 85%, and the yield of each product was as follows: CO₂ (49%), methane (2%), an oxygen-containing compound (18%), olefin (17%), and paraffin (7%). The total yield of the unsaturated hydrocarbon and the oxygen-containing compound was 37%, and the ratio of unsaturated hydrocarbon/oxygen-containing compound was 0.9.
In the hydrocarbon excluding the oxygen-containing compound, the olefin/paraffin ratio (hereinafter referred to as O/P) for C₂ to C₄ was 4; the O/P for C₅ to C₁₁ was 2; and the O/P for C₁₂ or higher was 1.
In the hydrocarbon excluding the oxygen-containing compound, the selectivity of each component was as follows: methane (8%), C₂ to C₄ (37%), C₅ to C₁₁ (45%), and C₁₂ or higher (10%).
Further, the selectivity of the main compounds, when all the oxygen-containing compounds were defined as 100, was as follows: methanol (8%), ethanol (57%), 1-propanol (16%), and 1-butanol (7%).

### Comparative Example 1

A catalyst was prepared in the same manner as in Example 2 except for using a manganese oxide support having a K-content of 8% by weight and an average pore size of 1 nm. The resulting catalyst was used to perform the FT reaction. However, the activity was lost in 1 hour after the reaction was started.

### Comparative Example 2

A silica support Cariact Q-50 manufactured by Fuji Silysia Chemical Ltd. (having a BET specific surface area of 76 m²/g, a pore volume of 1.30 ml/g, an average pore size of 58 nm, and a pellet size of 75 to 500 µm) in an amount of 5 g was impregnated simultaneously with an aqueous solution containing Fe(NO₃)₃·9H₂O in an amount corresponding to 20% by weight of the manganese oxide in terms of metallic iron and Cu(NO₃)₂·3H₂O in an amount corresponding to 3% by weight of manganese oxide in terms of metallic copper by the Incipient Wetness method using ultrasonic vibration. The resulting mixture was subjected to vacuum drying at 65°C for 6 hours, dried at 120°C for 12 hours, heated from room temperature to 400°C at 2°C/min, and calcined for 2 hours at 400°C.
The catalyst in an amount of 1 g prepared in this way was introduced into a slurry type reactor, and thereto were added 20 ml of PAO (poly-α-olefin). The reactor was controlled to a temperature of 280°C and a pressure of 1.0 MPa, and thereto was passed through a synthesis gas of H₂/CO=1/1 at 10 gh/mol for 6 hours to reduce the catalyst. The FT reaction was performed under the same conditions as in the reduction, and a sample was collected after the lapse of 10 hours and determined for products by GC using trans-decalin and 1-octanol as standard substances.
The CO conversion was 41%, and the yield of each product was as follows: CO₂ (39%), methane (1%), an oxygen-containing compound (1%), olefin (16%), and paraffin (7%). The total yield of the unsaturated hydrocarbon and the oxygen-containing compound was 17%, and the ratio of unsaturated hydrocarbon/oxygen-containing compound was 12.1.
In the hydrocarbon excluding the oxygen-containing compound, the O/P for C₂ to C₄ was 3; the O/P for C₅ to C₁₁ was 3; and the O/P for C₁₂ or higher was 0.5.
In the hydrocarbon excluding the oxygen-containing compound, the selectivity of each component was as follows: methane (5%), C₂ to C₄ (21%), C₅ to C₁₁ (62%), and C₁₂ or higher (12%).
Further, the selectivity of the main compounds, when all the oxygen-containing compounds were defined as 100, was as follows: methanol (17%), ethanol (43%), 1-propanol (17%), and 1-butanol (9%).

## Claims

1. A method for manufacturing an unsaturated hydrocarbon and an oxygen-containing compound, **characterized in that** the method comprises:
a first step of dispersing a catalyst in poly-α-olefin and reducing the catalyst with carbon monoxide or synthesis gas, wherein the catalyst is prepared by supporting iron on a support containing manganese and having an average pore size of 2 to 100 nm; and
a second step of bringing the catalyst after reduction in the first step into contact with synthesis gas under the conditions of a reaction temperature of 100 to 600°C and a reaction pressure of 0.1 to 10 MPa to obtain a reaction product containing an unsaturated hydrocarbon and an oxygen-containing compound.

2. The method for manufacturing an unsaturated hydrocarbon and an oxygen-containing compound according to Claim 1, **characterized in that** the reaction temperature in the second step is kept within the range of 280°C plus or minus 20°C.

3. The method for manufacturing an unsaturated hydrocarbon and an oxygen-containing compound according to Claim 1 or 2, **characterized in that** the catalyst is a catalyst prepared by further supporting copper and/or potassium on the support.

4. The method for manufacturing an unsaturated hydrocarbon and an oxygen-containing compound according to any one of Claims 1 to 3, **characterized in that** the support has an average pore size of 2 to 50 nm.

5. A catalyst **characterized in that** the catalyst is prepared by supporting iron on a support containing manganese and having an average pore size of 2 to 100 nm.

6. The catalyst according to Claim 5, **characterized in that** the catalyst is prepared by further supporting copper and/or potassium on the support.

7. The catalyst according to Claim 5 or 6, **characterized in that** the support has an average pore size of 2 to 50 nm.

8. A method for manufacturing a catalyst, **characterized in that** the method comprises:
a third step of mixing a support containing manganese and having an average pore size of 2 to 100 nm with a solution containing iron;
a fourth step of decompressing and drying the mixture obtained in the third step to allow the iron to adhere to the pores of the support to obtain a catalyst precursor; and
a fifth step of calcining the catalyst precursor obtained in the fourth step.
